# EUROPEAN PATENT SPECIFICATION

(11) **EP 4 082 527 B1**
(45) Date of publication and mention of the grant of the patent: **10.06.2026**
(21) Application number: 19957458.3
(22) Date of filing: 27.12.2019
(51) Int. Cl.: A61K 9/06, A61K 36/03, A61P 31/22, A61K 9/00, A61K 47/38

(54) **ANTIVIRAL COMPOSITION BASED ON EXTRACTS OF DURVILLAEA ANTARCTICA, USEFUL FOR TREATING LESIONS CAUSED BY ALPHAHERPESVIRINAE VIRUSES**
ANTIVIRALE ZUSAMMENSETZUNG AUF DER BASIS VON EXTRAKTEN VON DURVILLAEA ANTARCTICA ZUR BEHANDLUNG VON LÄSIONEN DURCH ALPHAHERPESVIRINAE-VIREN
COMPOSITION ANTIVIRALE À BASE D'EXTRAITS DE DURVILLAEA ANTARCTICA UTILE POUR TRAITER DES LÉSIONS PRODUITES PAR LE VIRUS ALPHAHERPESVIRINAE

(43) Date of publication of application: 02.11.2022
(73) Proprietor: Pontificia Universidad Católica De Chile, 8331150 Santiago (CL); Universidad de Concepción, 4070386 Concepción (CL)
(72) Inventor: GONZÁLEZ, Pablo, 8330025 Santiago (CL); AGURTO, Cristian, 4070409 Concepción (CL)
(74) Representative: Elzaburu S.L.P.
(86) International application number: PCT/CL2019/050159
(87) International publication number: WO 2021/127793

(56) References cited:
- FRANCISCA BOBADILLA ET AL: "Soluble &bgr;-1,3/1,6-glucan in seaweed from the southern hemisphere and its immunomodulatory effect", CARBOHYDRATE POLYMERS, vol. 92, no. 1, 1 January 2013 (2013-01-01), GB, pages 241 - 248, XP055347452, ISSN: 0144-8617, DOI: 10.1016/j.carbpol.2012.09.071
- ARRIETA M P ET AL: "Electrospun PVA fibers loaded with antioxidant fillers extracted fromDurvillaea antarcticaalgae and their effect on plasticized PLA bionanocomposites", EUROPEAN POLYMER JOURNAL, PERGAMON PRESS LTD OXFORD, GB, vol. 103, 12 April 2018 (2018-04-12), pages 145 - 157, XP085396916, ISSN: 0014-3057, DOI: 10.1016/J.EURPOLYMJ.2018.04.012
- LEONEL PEREIRA: "Electrospun PVA fibers loaded with antioxidant fillers extracted fromDurvillaea antarcticaalgae and their effect on plasticized PLA bionanocomposites", COSMETICS, vol. 5, no. 4, 22 November 2018 (2018-11-22), pages 68, XP055621542, DOI: 10.3390/cosmetics5040068
- ANONYMOUS: "DESARROLLO DE UNA FORMULACI�N ANTIVIRAL A PARTIR DE EXTRACTOS DE ALGAS PARDAS DEL LITORAI CHILENO, PARA TRATAR LESIONES CUTANEAS PRODUCIDAS POR VIRUS HERPES SIMPLE", 29 May 2019 (2019-05-29), XP055834566, Retrieved from the Internet <URL:https://gibmar.com/2019/05/29/conicyt-fondef-id17i-10143-2018-2019-desarrollo-de-una-formulacion-antiviral-a-partir-de-extractos-de-algas-pardas-del-litoral-chileno-para-tratar-lesiones-cutaneas-producidas-por-virus-her> [retrieved on 20210824]
- BARROS CAROLINE DE SOUZA ET AL: "Therapeutic efficacy in BALB/C mice of extract from marine algaCanistrocarpus cervicornis(Phaeophyceae) against herpes simplex virus type 1", JOURNAL OF APPLIED PHYCOLOGY, KLUWER, DORDRECHT, NL, vol. 29, no. 2, 18 May 2016 (2016-05-18), pages 769 - 773, XP036208900, ISSN: 0921-8971, [retrieved on 20160518], DOI: 10.1007/S10811-016-0865-9
- CASTILLO ESTEFAN�A ET AL: "Anti-herpetic Activity of Macrocystis pyrifera and Durvillaea antarctica Algae Extracts Against HSV-1 and HSV-2", FRONTIERS IN MICROBIOLOGY, vol. 11, 11 September 2020 (2020-09-11), XP093067668, DOI: 10.3389/fmicb.2020.02006
- ANONYMOUS: "DESARROLLO DE UNA FORMULACIÓN ANTIVIRAL A PARTIR DE EXTRACTOS DE ALGAS PARDAS DEL LITORAI CHILENO, PARA TRATAR LESIONES CUTANEAS PRODUCIDAS POR VIRUS HERPES SIMPLE", 29 May 2019 (2019-05-29), XP055834566, Retrieved from the Internet <URL:https://gibmar.com/2019/05/29/conicyt-fondef-id17i-10143-2018-2019-desarrollo-de-una-formulacion-antiviral-a-partir-de-extractos-de-algas-pardas-del-litoral-chileno-para-tratar-lesiones-cutaneas-producidas-por-virus-her>
- BARROS CAROLINE DE SOUZA; GARRIDO VALERIA; MELCHIADES VANESSA; GOMES RAFAELA; GOMES MAX WILLIAN; TEIXEIRA VALERIA LANEUVILLE; PAIX: "Therapeutic efficacy in BALB/C mice of extract from marine algaCanistrocarpus cervicornis(Phaeophyceae) against herpes simplex virus type 1", JOURNAL OF APPLIED PHYCOLOGY., KLUWER, DORDRECHT., NL, vol. 29, no. 2, 18 May 2016 (2016-05-18), NL, pages 769 - 773, XP036208900, ISSN: 0921-8971, DOI: 10.1007/s10811-016-0865-9
- PENG YAN, XIE ENYI, ZHENG KAI, FREDIMOSES MANGALADOSS, YANG XIANWEN, ZHOU XUEFENG, WANG YIFEI, YANG BIN, LIN XIUPING, LIU JUAN, LI: "Nutritional and Chemical Composition and Antiviral Activity of Cultivated Seaweed Sargassum naozhouense Tseng et Lu", MARINE DRUGS, vol. 11, no. 12, 1 January 2013 (2013-01-01), pages 20 - 32, XP055834565, DOI: 10.3390/md11010020
- SUN QI-LI; LI YI; NI LONG-QUAN; LI YI-XUAN; CUI YONG-SHENG; JIANG SI-LIANG; XIE EN-YI; DU JUAN; DENG FEI; DONG CAI-XIA: "Structural characterization and antiviral activity of two fucoidans from the brown algae Sargassum henslowianum", CARBOHYDRATE POLYMERS, APPLIED SCIENCE PUBLISHERS , LTD BARKING, GB, vol. 229, 20 October 2019 (2019-10-20), GB, XP085942662, ISSN: 0144-8617, DOI: 10.1016/j.carbpol.2019.115487
- PONCE NORA M.A.; FLORES MARÍA L.; PUJOL CARLOS A.; BECERRA MÓNICA B.; NAVARRO DIEGO A.; CÓRDOBA OSVALDO; DAMONTE ELSA B.; STORTZ C: "Fucoidans from the phaeophytaScytosiphon lomentaria: Chemical analysis and antiviral activity of the galactofucan component", CARBOHYDRATE RESEARCH, PERGAMON, GB, vol. 478, 1 January 1900 (1900-01-01), GB, pages 18 - 24, XP085691690, ISSN: 0008-6215, DOI: 10.1016/j.carres.2019.04.004
- SUDIPTA SAHA; MOJDEH HEIDARY NAVID; SHRUTI S. BANDYOPADHYAY; PAUL SCHNITZLER; BIMALENDU RAY;: "Sulfated polysaccharides from: Structural features andantiviral activities", CARBOHYDRATE POLYMERS, APPLIED SCIENCE PUBLISHERS , LTD BARKING, GB, vol. 87, no. 1, 19 July 2011 (2011-07-19), GB, pages 123 - 130, XP028316746, ISSN: 0144-8617, DOI: 10.1016/j.carbpol.2011.07.026

## Description

### Field of Invention

The present invention relates to a composition with antiviral activity, which comprises water-soluble alginate-free extracts of *Durvillaea antarctica.* The composition is useful for the treatment and prevention of viral infection produced by herpes virus of the subfamily *Alphaherpesvirinae.* Preferably, the composition of the invention is useful to prepare an antiviral drug for topical application intended for the treatment or prevention of skin or oral lesions caused by herpes virus of the subfamily*Alphaherpesvirinae.*

### Background to the Invention

Herpesviruses or viruses of the family *Herpesviridae* are prevalent in the human population, infect the host for life and can recur over time. This family of viruses is divided into three subfamilies. The subfamily *Alfaherpesvirinae* contains human herpes viruses 1, 2 and 3, which correspond to herpes simplex virus 1 (HSV-1 or HHV-1), herpes simplex virus 2 (HSV-2 or HHV-2) and varicella zoster virus (VZV or HHV-3). The subfamily *Betaherpesvirinae* contains human herpes viruses 5, 6 and 7, which correspond to cytomegalovirus (CMV, HHV-5), sudden rash virus (HHV-6) and HHV-7. The subfamily *Gammaherpesvirinae* contains human herpes viruses 4 and 8, which correspond to Epstein-Barr viruses (EBV, HHV-4) and Kaposi's sarcoma virus (KSV, HHV-8), respectively.

Viruses of the subfamily *Alphah*erpesvirinae share important similarities, such as large doublestranded DNA genomes, homologous genes, the presence of an integument in the virion, replication processes, the ability to produce persistent infection and establish latency in the host. Some of these viruses are herpes simplex virus type 1 (HSV-1), herpes simplex virus type 2 (HSV-2) and varicella zoster virus (VZV) which affect the general population with high prevalence.

Herpes simplex viruses of types 1 and 2 produce a wide spectrum of diseases that can be of a serious nature, such as encephalitis, compromising the life of the individual, or of a non-dangerous nature, but of high morbidity. Frequent manifestations of infections by HSV-1 virus, HSV-2 are the development of skin lesions evident on the skin and sometimes painful, both in the oro-facial area (*herpes labialis,* usually HSV-1), the genital area (*herpes genitalis,* usually HSV-2), torso (*eczema herpeticum, herpes gladiatorium* by HSV-1, HSV-2 or VZV) or fingers (herpetic panadizo, usually HSV-1) that, although they do not endanger the life of the individual, they do generate symptoms that are annoying (unsightly and sometimes painful) and that can affect the quality of life of people and the work performance of individuals. These lesions can last up to 3 and 6 weeks in the context of primary infections for HSV-1 and HSV-2, respectively and up to 7-10 days in the case of subsequent recurrences, which occur throughout the life of the individual because they are persistent viruses that establish latent infection for life in people. Herpes simplex viruses can also cause herpetic gingivostomatitis, which consists of infection and inflammation of the gums, and is usually caused by HSV-1 infection in young children. Since this herpetic manifestation occurs frequently in children under 12 years of age, it cannot be treated with the most common antivirals currently commercially available for these viruses, as these are indicated for individuals over 12 years of age. In addition, herpes simplex viruses can cause eye complications, mainly HSV-1, such as conjunctivitis and epithelial or stromal herpetic keratitis, which can lead to permanent blindness. On the other hand, VVZ also frequently produces skin manifestations producing during the primary infection a rash similar to blisters on the skin throughout the body that gives itching and then in recurrent infections herpes zoster that mainly affects the torso and face producing extensive and painful lesions on the skin.

An important aspect of herpes simplex and varicella zoster viruses, like other herpes viruses, is their ability to infect people for life, being able to establish persistent infection in the nerve tissues of the individual. From these sites, these viruses can be reactivated as a result of various stimuli, such as UV radiation, hyperthermia and hypothermia, among others, in addition to unknown causes to produce recurrent pathology throughout the life of the individual. Currently, it is estimated that between 10-25% and 0.4% of individuals infected with herpes simplex virus and varicella zoster virus, respectively manifest symptoms of the disease, particularly skin lesions or mucous membranes in various forms (*herpes labialis, herpes genitalis, eczema herpeticum, herpes gladiatorium,* shingles, herpetic panadizo, herpetic keratitis and herpetic gingivostomatitis). With this, up to 16% of the world's human population will manifest herpetic lesions product of these viruses of the subfamily *Alphaherpesvirinae.* This high percentage of the population affected by the symptoms of these viruses raises a significant number of individuals who can potentially make use of new effective solutions to address the health problems produced by these pathogens.

While there are commercially available antivirals to treat skin lesions by viruses of the subfamily *Alphahehrpesvirinae,* the most commonly used being the nucleosides Acyclovir and Valacyclovir, these are generally ineffective. In the case of herpes simplex virus these antivirals usually only reduce by approximately 1-2 days, from a total of 7-10 days, the recovery time of the lesions and in some cases their effects are imperceptible to some individuals. In the case of shingles produced for VVZ, this same type of drugs reduce by less than 50% the residual pain after 6 months of the viral clinical manifestation.

On the other hand, approximately 3.5-10% of immunosuppressed individuals (those with transplants, treated for autoimmune diseases or HIV+) develop infections with HSV variants that are resistant to the most commonly used "first-line" antivirals (e.g. Acyclovir and derived molecules). Unfortunately, the most commonly available pharmaceutical alternatives today for these cases frequently produce numerous adverse effects on the patient that may require supervision and medical attention. Although, in the case of immunocompetent individuals, these resistant HSV variants occur only in approximately 1% of cases, given the high prevalence of these viruses in the population, the number of individuals affected with this type of drug-resistant virus is significant.

On the other hand, there are second-generation drugs used to treat herpes simplex virus infections that are resistant to Acyclovir. These drugs are mainly: Cidofovir and Foscarnet, drugs that inhibit viral DNA polymerase dependent DNA and do not require viral proteins (i.e. viral TK) for activation in the cell. Although these drugs are effective against HSV variants resistant to Acyclovir, the adverse side effects they produce are numerous and occur in a large percentage of individuals. Some of these side effects can be nephrotoxicity, neutropenia, myelosuppression, confusion, altered mental state, hallucinations, nightmares, anxiety, ataxia, tremors, seizures, fever, abnormal levels of liver enzymes in serum, diarrhea and nausea, among others.

Another alternative available to treat herpes simplex virus is a combination of Acyclovir and topical hydrocortisone. This combination reduces the duration of herpetic lesions by 1.6 days (compared to 1.0 days for Acyclovir applied topically alone) and reduces the size of the lesion area by 50%. Although this strategy constitutes a statistically significant improvement for the treatment of herpetic lesions, it still evidences the need to identify drugs or combinations of drugs that have better effectiveness.

In the state of the art prior to this invention there are some publications that are close to the invention, which are briefly summarized below.

In the publication by Souza Barros, Caroline, et al. "Therapeutic efficacy in BALB/C mice of extract from marine algae Canistrocarpus cervicornis (Phaeophyceae) against herpes simplex virus type 1" Journal of Applied Phycology 29.2 (2017): 769-773, an extract with antiviral properties is obtained from a brown algae, such as *Canistrocarpus cervicornis ,* and the extract obtained is used for the treatment of HSV-1 skin lesions. The invention differs from this document, in that the algae is distinct and uses a protein fraction, while in the publication it is indicated that the action is given by diterpenes, which may present toxicity, and the document only suggests that it has a low toxicity because no changes in body weight, or in liver or kidney function are observed in the 16 days of the study. It is also said that results are observed on day 10, with 3 daily applications of the extract.

Other documents of the state of the art include:
Francisca Bobadilla et al: ("Soluble β-1,3/1,6-glucan in seaweed from the southern hemisphere and its immunomodulatory effect",CARBOHYDRATE POLYMERS,vol. 92, no. 1, 1 January 2013 pages 241-248), discloses a process for the preparation of a water soluble extract from *Durvillaea antarctica* with 85% ethanol as solvent to recovery β-1,3/1,6-glucan polysaccharide. The crude extract is treated with CaCl₂, which leads to a precipitation of alginates. They study of the immunomodulatory activity of the soluble β-1,3/1,6-glucan polysaccharide obtained from macroalgae in the southern hemisphere, specifically from the species *Durvillaea antarctica.* No antiviral activity is mentioned.

Arrieta M P et al ("Electrospun PVA fibers loaded with antioxidant fillers extracted fromDurvillaea antarcticaalgae and their effect on plasticized PLA bionanocomposites", EUROPEAN POLYMER JOURNAL, PERGAMON PRESS LTD OXFORD, GB,vol. 103, 12 April 2018 (2018-04-12), pages 145-157) discloses aqueous and aqueous ethanolic extraction of antioxidant polyphenols from *Durvillaea antarctica.* In this document alginates are not removed from the extract and no antiviral activity is mentioned.

Leonel Pereira ("Electrospun PVA fibers loaded with antioxidant fillers extracted fromDurvillaea antarcticaalgae and their effect on plasticized PLA bionanocomposites", COSMETICS, vol. 5, no. 4, 22 November 2018 (2018-11-22), page 68) reviews cosmetic and pharmaceutical uses of *Durvillaea antarctica* extracts for skin care. An undefined extract from *D. antarctica* is mentioned for skin moisturization and protection.

Anonymous: ("DESARROLLO DE UNA FORMULACIÓN ANTIVIRAL A PARTIR DE EXTRACTOS DE ALGAS PARDAS DEL LITORAI CHILENO,PARA TRATAR LESIONES CUTANEAS PRODUCIDAS POR VIRUS HERPES SIMPLE", 29 May 2019 (2019-05-29)), discloses the antiviral activity of brown seaweeds against herpes simplex viruses. This corresponds to an online publication of the abstract of the project of the inventors before developing the invention. It is mentioned that algae from the Chilean coast display antiviral activity against herpes simplex virus, however no specific extract is mentioned.

The current state of the art for the treatment or prevention of skin infections caused by viruses of the subfamily *Alphahehrpesvirinae* (for example HSV-1, HSV-2 and VZV) requires the development of new antiviral formulations that give better results of resolution time and pain reduction than current alternatives and that at the same time have low side effects.

The invention solves this need by contributing to the state of the art, a new composition obtained from water-soluble alginate-free extracts of *Durvillaea antarctica* algae with antiviral properties. The antiviral activity is particularly high in its protein fraction, especially useful for to prepare an antiviral drug, that allows to prevent or treat skin or oral lesions caused by herpes virus of the subfamily *Alphahehrpesvirinae.*

Where *Durvillaea antarctica* is an edible algae, so its use is safe, and therefore the extracts of the invention have the additional advantage of being of natural origin, from a renewable source such as algae, where its production does not require chemical synthesis processes, so they do not have the danger of dragging potentially toxic reaction by-products for the human or animal body.

### Description of the Figures

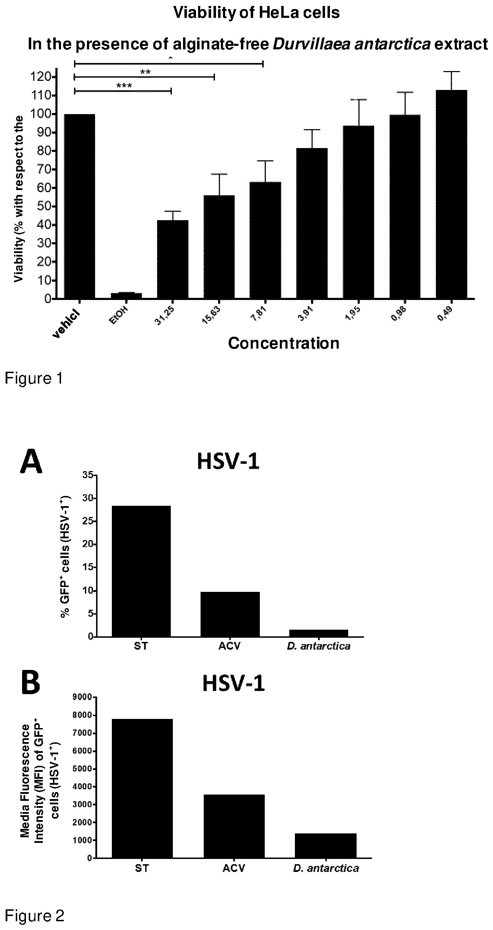

**Figure 1****:** Cell viability assay. HeLa cells were continuously treated with water-soluble extract of alginate-free *Durvillaea antarctica* at concentrations 0.49-125 mg/mL for 24 hours and then incubated for one hour at 37°C with AlamarBlue^{®} (resazurin) and quantified by colorimetry on black wall plates and transparent background. The figure shows the percentage of viable cells for each extract concentration. As controls, cells treated with vehicle (saline phosphate, PBS) and cells treated with 75% ethanol (lethal) are incorporated. Significant cell death values were obtained for any concentration greater than 7.81 mg/ml by one-way ANOVA (*p <0.05; **p <0.01; ***p <0.001). The CC50 calculated by linear regression is 25 mg/ml.

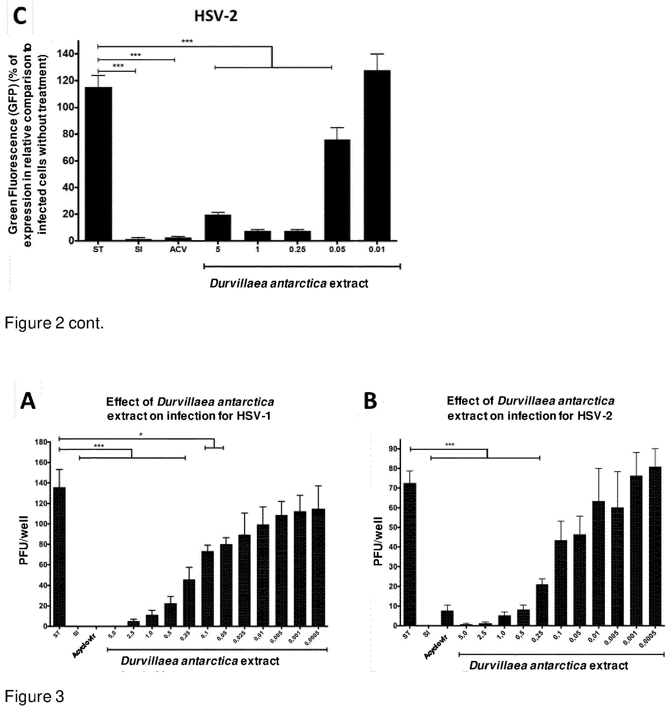

**Figure 2****:** Expression of the green fluorescent protein gene (GFP reporter) in HeLa cells infected with A-B. HSV-1 in the presence of 1 mg/ml of alginate-free water-soluble extract of *Durvillaea antarctica,* or C. HSV-2 in the presence of different concentrations (0.01, 0.05; 0.1, 0.25 and 5 mg/mL) of water-soluble extract of *Durvillaea antarctica* free of alginates . In A. the percentage of positive GFP cells (HSV-1 positive) is shown and in B. the fluorescence intensity associated with GFP (HSV-1) 24 hours post-infection determined by flow cytometry. In C. the fluorescence intensity of GFP (VHS-2) per well determined with a multi-mode reader is shown. Controls: cells with infection but no treatment (TS), cells without infection and without treatment (SI), cells treated with 50 ug/ml of Acyclovir). Statistical analysis performed by one-way ANOVA (***p <0.001).
**Figure 3****:** Production of plaque forming units (PFUs) in HeLa cells incubated with different concentrations of water-soluble extract of *Durvillaea antarctica* free of alginates of the invention treated continuously to viral inoculation with a known amount of PFU of A. HSV-1 or B. VHS-2. The number of PFUs was quantified 24 hours after infection. A group without treatment but with infection (TS), a group without treatment without infection (SI) and treatment with Acyclovir (50 ug/ml) are included as controls. Significant values of antiviral effectiveness were obtained for any concentration greater than 0.05 mg/ml for HSV-1 and 0.25 mg/ml for HSV-2 by one-way ANOVA (*p <0.05; ***p<0.001). The EC50 calculated by linear regression is 0.13 mg/ml for HSV-1 and 0.15 mg/ml for HSV-2.

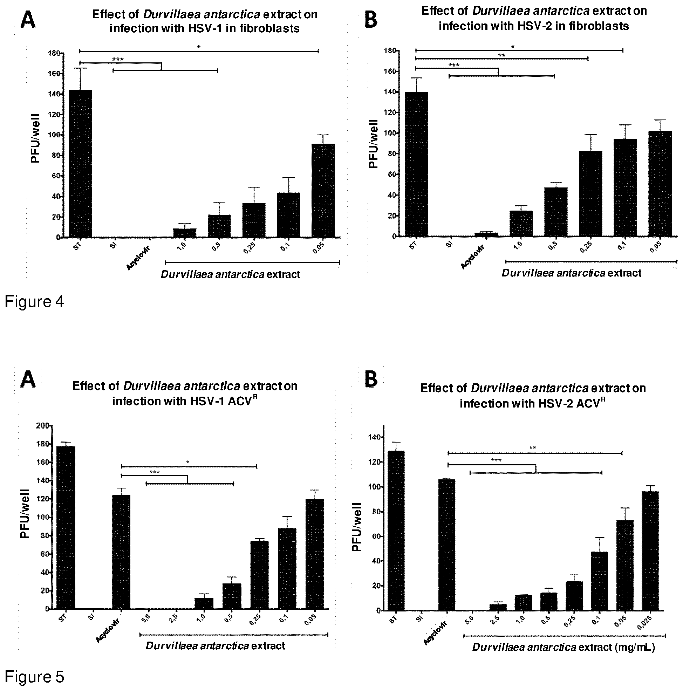

**Figure 4****:** Production of plaque forming units (PFUs) in oral gingival fibroblasts incubated with different concentrations (mg/ml) of water-soluble extract of *Durvillaea antarctica* free of alginates of the invention treated continuously to viral inoculation with a known amount of PFU of A. HSV-1 or B. VHS-2. The number of PFUs was quantified 24 hours after infection. Included as controls are a group without treatment but with infection (TS), a group without treatment without infection (SI) and treatment with Acyclovir (50 ug/ml). Significant values of antiviral effectiveness were obtained for any concentration greater than 0.05 mg/ml for HSV-1 and 0.1 mg/ml for HSV-2 by one-way ANOVA (*p <0.05; **p <0.01; ***p <0.001).
**Figure 5****:** Production of plaque forming units (PFU) in HeLa cells incubated with different concentrations (mg/ml) of water-soluble extract of *Durvillaea antarctica* free of alginates of the invention treated continuously to viral inoculation with a known amount of PFU of HSV-1 or HSV-2 resistant to Acyclovir (ACV^{R}). The number of PFUs was quantified 24 hours after infection. Included as controls are a group without treatment but with infection (TS), a group without treatment without infection (SI) and treatment with Acyclovir (50 ug/ml). Significant values of antiviral effectiveness were obtained for any concentration greater than 0.25 mg/ml for HSV-1 and 0.05 mg/ml for HSV-2 by one-way ANOVA (*p <0.05; **p <0.01; ***p <0.001).

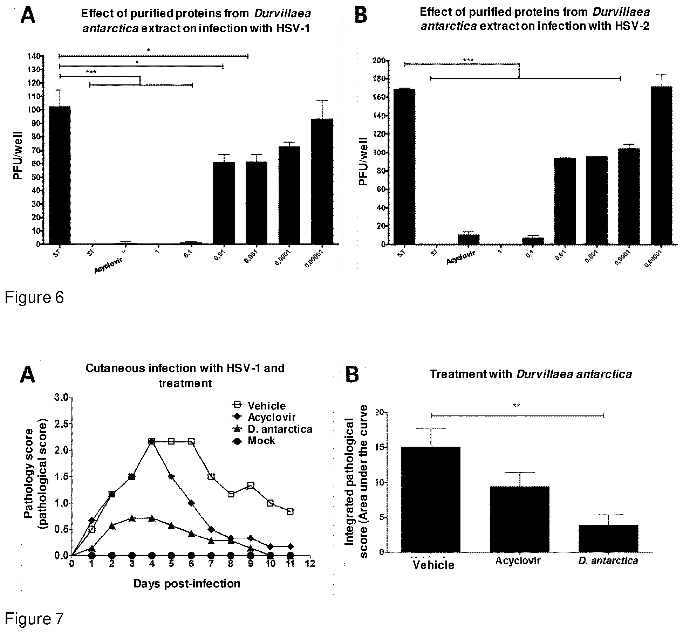

**Figure 6****:** Production of plaque forming units (PFUs) in heLa cells incubated with different concentrations (mg/ml) of proteins precipitated from water-soluble extract of Alginate-free *Durvillaea antarctica* which were continuously treated with the protein fraction related to viral inoculation, which was performed with a known amount of PFU of A. HSV-1 or B. HSV-2. The number of PFUs was quantified 24 hours after infection. Included as controls are a group without treatment but with infection (TS), a group without treatment without infection (SI) and treatment with Acyclovir (50 ug/ml). Significant values of antiviral effectiveness were obtained for any concentration greater than 0.001 mg/ml for HSV-1 and 0.0001 mg/ml for HSV-2 by one-way ANOVA (*p <0.05; **p <0.01; ***p <0.001).
**Figure 7****:** A. Evaluation of pathological progression associated with HSV-1 skin infection for 11 days. Clinical manifestations produced in the skin of animals (mice, *mus musculus*) infected with HSV-1 and treated 2 times daily with *alginate-free Durvillaeae antarctica* extract of the invention formulated pharmaceutically with hydroxymethylcellulose in a concentration 5 mg/ml or vehicle were evaluated, only topically. Included as a control is a group treated with Acyclovir in 5% cream format (commercial, bioequivalent) after infection with HSV-1 and a group of animals that was prepared for infection (hair removal and skin erosion), but without infection (Mock). B. Quantification of the area under the curve (AUC) of the data shown in A (except Mock which has values tending to zero)," which integrates the severity and duration of the infection (score and time). One-way ANOVA (**p <0.01).

### Detailed description of the invention

The present invention provides a composition comprising an extract prepared from *Durvillaea antarctica,* where the extract corresponds to the water-soluble fraction and is substantially free of the gelling agent alginates. This composition has antiviral properties, and is especially useful for to prepare an antiviral drug to prevent or treat skin or oral lesions caused by herpes virus of the subfamily *Alphahehrpesvirinae (*e.g. HSV-1, HSV-2 and VZV), both sensitive and resistant to Acyclovir.

The inventors have developed a composition based on water-soluble extracts of *Durvillaea antarctica,* where the extract obtained is substantially free of alginates, a compound with gelling properties. This extract can be used completely, or only its protein fraction, since this fraction maintains the highest activity. The proteins with antiviral activity have not yet been fully identified by the inventors.

It is important to note that the invention mainly targets the water-soluble or protein fraction, which differs from organic fractions, where high concentrations of organic polymers such as alginates, fucoidans and phenolic compounds, among others, are obtained.

Thus the invention points to a composition with antiviral activity comprising water-soluble extracts of *Durvillaea antarctica* substantially free of alginates and optionally a carrier or vehicle. Where the carrier or vehicle may simply be water, buffer, saline, ethanolic solution, or excipients for formulation of pharmaceutically acceptable hydrogels, creams or ointments, such as carboxymethylcellulose, hydroxymethylcellulose, glycerol, propylene glycol or others available in the technique, optionally together with preservatives or stabilizers.

Where the water-soluble extract of *Durvillaea antarctica* substantially free of alginates or their protein fraction is present in a concentration between 0.05 to 50 mg/mL. Especially between 0.1 to 25 mg/mL, and more especially preferred between 1 and 10 mg/mL.

In a second aspect, the invention refers to the process of preparation of a composition, which includes the following steps:
(a) mixing ground biomass of *Durvillaea antarctica* with water in a ratio of 1 to 5 % w/v, at a temperature between 15 and 45°C and stirring for 2 to 24 hours;
(b) separating the residual biomass from the water-soluble supernatant;
c) removing the alginates present in solution, obtaining the water-soluble phase, which constitutes the extract which is optionally mixed with a carrier or vehicle.

Where in step (b) the biomass is separated by centrifugation or filtration, or any other available technique. And in step (c) the alginates are removed for example by precipitation with absolute ethanol in a ratio of 1:1 ethanol to extract, and then centrifuged or filtered to separate the precipitated alginates, or by any other technique available for this purpose.

In one embodiment the added ethanol can be removed by evaporation, especially by evaporation at reduced pressure, for example, at 175 mBar. Once the ethanol is removed, the extract of the invention is available and which can be used directly, or optionally can be frozen and freeze-dried, to then be resuspended in water or pH 7 buffer.

Optionally, a precipitation of the proteins in the extract is carried out, to obtain the protein fraction, for example, by precipitation with ammonium sulfate in a ratio of 1:1 ammonium sulfate to extract. Where this protein fraction maintains the greatest effectiveness of the extract of the invention. In this way the extract of the invention can be used in different degrees of purification or fractionation, maintaining its activity, where all these variants are part of the present invention. Finally, the invention points to the use of the composition containing water-soluble extracts of *Durvillaea antarctica* substantially free of alginates or their protein fraction, to prepare an antiviral drug. Where in a preferred embodiment the drug is for topical application for skin and mucous membranes. This medicine helps prevent or treat skin lesions caused by viruses of the subfamily *Alphaherpesvirinae* (e.g. HSV-1, HSV-2 and VZV), sensitive or resistant to Acyclovir.

The antiviral composition of the invention can be used to relieve the symptoms of herpes virus infection and/or to improve the aesthetics of skin or oral lesions produced by herpes simplex virus or varicella zoster virus.

Surprisingly, the inventors have found that the extracts of *Durvillaea antarctica,* as described in this invention, decrease by approximately 96% and 94% the infection of HSV-1 and HSV-2 viruses, respectively on human cells (HeLa cells at 2.5 mg/ml) in *in vitro* experiments.

The inventors have performed tests to determine the selectivity index (SI) of the composition of the *invention in vitro,* based on tests of reduction in the number of plate-forming units (PFU) after viral infection and treatment. This index corresponds to the value obtained from the CC50/EC50 ratio. The higher the value of the CC50/EC50 ratio (>1), the greater the therapeutic potential of antiviral compounds, since it means that they have high antiviral activity, over adverse effects on the viability of host cells (substrate cells).

On the one hand, the CC50 value (cytotoxic concentration 50%) in culture is calculated, which corresponds to the concentration of algae extract that causes the death of 50% of host cells in the culture. On the other hand, the EC50 value (effective concentration 50%) is calculated to determine the concentration of extract that inhibits 50% of viral activity. These assays are shown in example 6, included below, and gave for the alginate-free *durvillaea antarctica* extract of the invention an approximate value of 192 for HSV-1 and 167 for HSV-2 (based on results of viability and effectiveness in HeLa cells). For reference, the selectivity index of Acyclovir, the most commonly used drug to treat herpes virus skin lesions, is 300-340.

The inventors also developed *in vivo* tests, where the composition of the invention significantly decreased the severity of the injury caused by HSV-1, and decreased the time and severity of infection with respect to the untreated control, which is exposed as the integration of these two parameters (area under the curve, AUC). It is significant to indicate that Acyclovir, although it reduced the injury time was less effective in decreasing its severity compared to the alginate-free *Durvillaea antarctica* extract of the invention. The AUC of Acyclovir is greater than the AUC of the alginate-free extract of the invention

For the expert in the technique it will be evident that the antiviral properties of the composition of the invention, although they have been demonstrated in the examples for the HSV-1 and HSV-2 viruses, are extrapolable to other viruses of the subfamily *Alphahehrpesvirinae,* for example, VZV.. As demonstrated in the examples included below, the invention shows an equivalent effectiveness *in vitro* in inhibiting the production of infectious plaques of herpes simplex virus, sensitive or resistant to Acyclovir, making it especially useful for the control of the latter viruses.

Examples are described below to better understand the invention.

### Examples.

### Example 1: Obtaining the extract of D. antarctica.

Specimens of the macroalgae *Durvillaea antarctica* collected in Caleta Chome (Biobío Region, Chile) were washed with drinking water for the elimination of mineral salts, sand and epiphytic organisms. Subsequently, the macroalgae were freeze-dried for 5 days at a temperature of - 70°C±1°C, ground and sieved in a 0.5 mm sieve. The sieved dry biomass powder was stored in vacuum-sealed plastic bags.

Subsequently, 3.00 g of freeze-dried biomass was measured and taken to an Erlenmeyer flask. 120 mL of deionized water was added and kept in agitation, in an orbital agitator, at 200 RPM at a temperature of 30°C for 4 h.

It was then centrifuged at 4,000 RPM for 10 min and the decanted residual biomass was removed. Obtaining the water-soluble extract of the algae, which must be subjected to additional purifications to eliminate alginates and wall polysaccharides from the algae, which may be present.

Alginate precipitation was performed by adding absolute ethanol to the crude water-soluble extract of *Durvillaea antarctica* in a 1:1 ratio. Due to the change in polarity of the solution of extracts, alginates precipitated.

Subsequently, to achieve a good separation of the alginates, the dissolution of algal extracts with ethanol was centrifuged at 4,000 RPM for 10 min obtaining the water-soluble extract substantially free of alginates.

Finally, ethanol was removed from the water-soluble phase of the extract by evaporation of ethanol at reduced pressure (175 mBar).

### Example 2: Cytotoxicity test.

To assess whether *Durvillaea antarctica* extract has toxic effects on human cells, a cell viability assessment was performed after being incubated in the presence of different concentrations of the alginate-free extract of *Durvillaea antarctica* obtained in Example 1.

Continuous treatments were performed on HeLa cells with the alginate-free *Durvillaea antarctica* extract at concentrations of 0.1-125 mg/mL for 24 hours. Subsequently, the cells were incubated for one hour at 37°C with AlamarBlue^{®} (reagent based on resazurin), and then evaluated by a colorimetric quantification assay the percentage of viable cells for each condition compared to the control without treating with the extract. The results are shown in Figure 1, and show that there was incremental toxicity of the alginate-free extract of the invention on human cells at concentrations greater than 7.81 mg/mL.

### Example 3: Evaluation of antiviral activity of water-soluble extracts in vitro by evaluating the expression of a reporter gene encoded in HSV genomes.

To evaluate the antiviral activity of the alginate-free water-soluble extracts of the invention, obtained in example 1, HeLa cells (ATCC^{®} CCL-2^{™}, human cervix epithelial cells) were used as target cells to analyze HSV-1 and HSV-2 infection. These cells have the advantage of being human and therefore it is expected that the results obtained in this model resemble what would happen in a physiological context of natural infection.

HeLa cells were treated with water-soluble extract of alginate-free *Durvillaea antarctica* obtained in example 1, prior to infection with varying extract concentrations (mg/ml) and inoculated with known amounts of plaque-forming units (PFUs) of HSV-1 K26GFP or HSV-2 ZAG(333), viral strains that contain in their genome the gene that codes for green fluorescent protein (GFP).

After 24 hours post-infection, the number of green fluorescence-positive cells derived from GFP expression (HSV-1) or the green fluorescence intensity derived from GFP expression (HSV-2) were quantified by flow cytometry or in a multimode plate reader, respectively. Figure 2 shows the results, where the data is shown, expressed as the number of green fluorescence positive cells derived from GFP expression (HSV-1) or the green fluorescence intensity derived from GFP expression (HSV-2) as quantified by flow cytometry or in a multimode plate reader, respectively. The extract of the invention resulted in a decrease of more than 95% of the fluorescence of HSV-1 GFP and more than 91% of the fluorescence of HSV-2 GFP for the condition of treatment with 1 mg/ml of water-soluble extract of alginate-free *Durvillaea antarctica.*

### Example 4: Inhibition of infectious plaque production.

HeLa cells were treated with different concentrations (mg/mL) of water-soluble extract of alginate-free *Durvillaea antarctica,* obtained in example 1, continuously with respect to the challenge with HSV with a known amount of HSV-1 K26GFP or HSV-2 ZAG(333). Within 24 hours of infection, the number of plaque forming units (PFUs) present in each well was determined. The results are shown in Figure 3, where it is seen that the treatment with water-soluble extract of alginate-free *Durvillaea antarctica* of the invention decreases the production of PFUs in the cultivation.

A trial similar to the previous one was performed, but on another cell type, healthy human oral gingival fibroblasts, to evaluate the response in the oral cavity, where herpes virus infections frequently occur. The results are shown in Figure 4, where a similar result of the water-soluble extract of alginate-free *Durvillaea antarctica* obtained in example 1 on these human primary cells infected with HSV-1 or HSV-2 is observed.

On the other hand, a test was carried out in a similar way to what has already been described, on HeLa cells, which were incubated with different concentrations (mg/ml) of water-soluble extract of alginate-free *Durvillaea antarctica* obtained in example 1, continuously with respect to viral inoculation, with a known amount of PFU of HSV-1 or HSV-2 resistant to Acyclovir (ACV^{R}).

The results show that the extract of the invention is able to decrease the production of PFUs in the cultivation, see Figure 5, and therefore, the water-soluble extract of alginate-free *Durvillaea antarctica* of the invention is effective against HSV-1 or VHS-2 (ACV^{R}). As in previous trials (Figures 3 and 4), the number of PFUs was quantified 24 hours after infection.

Treatments with Acyclovir (50 ug/ml), group without treatment but with infection (TS) and group without treatment without infection (SI) were included as controls in all these experiments.

### Example 5: Selectivity Index (IS).

### 5.1 Cytotoxic concentration 50% (CC50)

To determine cell viability after the addition of increasing concentrations of alginate-free algae extracts, the cytotoxic concentration 50% (CC50) of the water-soluble extract of alginate-free *Durvillaea antarctica* was determined, obtained according to Example 1. The viability of HeLa cells was evaluated 24 hours after being treated with increasing concentrations of the extract of the invention between 0.49 to 31.25 mg/ml, as described in example 2. The results of Figure 1 allow the calculation of the concentration of extract that induces cell viability 50% (CC50), this being 25 mg/mL.

### 5.2 Effective concentration 50% (EC50)

The effective concentration 50% (EC50) antiviral of the water-soluble extract of alginate-free *Durvillaea antarctica* of the invention corresponds to the formation of plaque-forming units (PFU) after the continuous addition of the extract with respect to the challenge with HSV and then a known amount of HSV-1 K26GFP or HSV-2 ZAG(333) 24 hours after infection, evaluated in example 4. The results shown in Figure 3 allow the calculation of that treatment with 0.13 mg/ml and 0.15 mg/ml of water-soluble extracts of alginate-free *Durvillaea antarctica* decreases by 50% the production of HVS-1 and HSV-2 PFUs in the culture, respectively, these being the EC50 values for the extract with each type of virus.

### 5.3 Selectivity Index (SI)

Based on the data obtained from CC50 and EC50 in HeLa cells described above for HSV-1 and HSV-2, it is possible to determine approximate selectivity index values (CC50/EC50) of 192 and 167 for HSV-1 and HSV-2, respectively with the water-soluble extract of *Durvillaea antarctica* of the invention.

### Example 6: Antiviral activity against herpes simplex virus in fractions of the water-soluble, alginate-free Durvillaea antarctica extract.

To determine the nature of the antiviral activity in the water-soluble extracts of alginate-free *Durvillaea antarctica* of the invention a precipitation of proteins was made from the water-soluble, alginate-free *Durvillaea antarctica* extract obtained in example 1 with ammonium sulfate in a 1:1 weight/volume propotion and then these were resuspended in saline buffer solution (PBS) and their antiviral activity was evaluated.

As shown in Figure 6, proteins precipitated from the water-soluble, alginate-free *Durvillaea antarctica* extract exhibit significant antiviral activity against HSV-1 and HSV-2, at a lower concentration than the alginate-free parent extract, indicating that the ability to inhibit the replication of herpes simplex virus in the extract is mainly due to its protein component.

### Example 7: Formulation for topical application.

The extract of the invention obtained in example 1 was incorporated at a final concentration of 5 mg/mL, in a pharmaceutical base of hydrogel composed of hydroxymethylcellulose. The resulting formulation can be used directly on a skin lesion to reduce the adverse effects of HSV-1 skin infection.

### Example 8: Antiviral activity in vivo.

For the ability of the water-soluble, alginate-free *Durvillaea antarctica* extract of the invention to inhibit the progression of pathological damage after skin infection by HSV-1, animals (mice) were infected with HSV-1 and followed for 11 days, being treated 2 times a day, 24 h after infection, either with the composition of the invention (formulation for topical application obtained in example 7, or Acyclovir cream 5% for clinical use (commercially available, bioequivalent). In Figure 7, it is shown that the treatment with the formulation contained in the invention reduces the pathology significantly compared to the group treated with the vehicle when considering, both severity and duration of the pathology (integrated with the value of the area under the curve of the graphs, AUC) (Figures 7A and 7B). Treatment with the water-soluble extract of alginate-free *Durvillaea antarctica* of the invention gave a better *in vivo* result than treatment with 5% Acyclovir bioequivalent in cream.

## Claims

1. A composition with antiviral activity **CHARACTERIZED in that** it comprises water-soluble alginate-free extracts of *Durvillaea antarctica,* in a concentration between 0.05 to 50 mg/mL and a carrier or vehicle.

2. The composition according to claim 1 **CHARACTERIZED in that** the water-soluble alginate-free extract of *Durvillaea antarctica,* corresponds only to the protein fraction of the extract.

3. Process of preparation of a composition with antiviral activity **CHARACTERIZED in that** it includes the following steps:
a) mixing ground biomass of *Durvillaea antarctica* with water in a ratio of 1 to 5 % w/v, at a temperature between 15 and 45°C and stirring for 2 to 24 hours;
b) separating the residual biomass from the water-soluble supernatant;
c) removing the alginates present in solution obtaining the water-soluble phase, which constitutes the extract which is optionally mixed with a carrier or vehicle.

4. Process according to claim 3 **CHARACTERIZED in that** in step b the biomass is separated by centrifugation or filtration.

5. Process according to claim 3 **CHARACTERIZED in that** in step c the alginates are removed by precipitation with absolute ethanol in a ratio of 1:1 ethanol to extract, and then centrifuged or filtered to separate the precipitated alginates.

6. Process according to claim 5 **CHARACTERIZED in that** ethanol is eliminated by evaporation.

7. Process according to claim 6 **CHARACTERIZED in that** optionally the alginate-free extract is freeze-dried.

8. Process according to claim 7 **CHARACTERIZED in that** the freeze-dried alginate-free extract is resuspended when used in water or buffer solution at pH 7.

9. Process according to claim 3 **CHARACTERIZED in that** additionally a fractionation is carried out, separating the protein fraction by using ammonium sulfate in a ratio of 1:1 weight/volume.

10. Composition according to claims 1 or 2 for use in the treatment and prevention of viral infection.

11. Composition for use according to claim 10, wherein the composition is used to prepare an antiviral drug.

12. Composition for use according to claim 11 wherein the drug is for topical application.

13. Composition for use according to claim 11 wherein the drug allows to prevent or treat skin or oral lesions caused by herpes virus of the subfamily *Alphaherpesvirinae*

14. Composition for use according to claim 11 wherein the drug allows to improve the aesthetics of skin or oral lesions caused by herpes virus of the subfamily *Alphaherpesvirinae*

15. Composition for use according to claims 13 and 14 **CHARACTERIZED in that** viruses of the subfamily *Alphaherpesvirinae* are selected between herpes simplex virus type 1 (HSV-1), herpes simplex virus type 2 (HSV-2) and varicella zoster virus (VZV).

## Patentansprüche

1. Eine Zusammensetzung mit antiviraler Aktivität, **DADURCH GEKENNZEICHNET, dass** sie wasserlösliche alginatfreie Extrakte von *Durvillaea antarctica* in einer Konzentration zwischen 0,05 bis 50 mg/ml und einen Träger oder ein Vehikel enthält.

2. Die Zusammensetzung nach Anspruch 1, **DADURCH GEKENNZEICHNET, dass** der wasserlösliche alginatfreie Extrakt von *Durvillaea antarctica* nur der Proteinfraktion des Extrakts entspricht.

3. Verfahren zur Herstellung einer Zusammensetzung mit antiviraler Aktivität, **DADURCH GEKENNZEICHNET, dass** es die folgenden Schritte einschließt:
a) Mischen von gemahlener Biomasse von *Durvillaea antarctica* mit Wasser in einem Verhältnis von 1 bis 5 % (w/v) bei einer Temperatur zwischen 15 und 45 °C und Rühren für 2 bis 24 Stunden;
b) Abtrennen der Restbiomasse vom wasserlöslichen Überstand;
c) Entfernen der in Lösung vorliegenden Alginate, um die wasserlösliche Phase zu erhalten, die den Extrakt darstellt, der optional mit einem Träger oder Vehikel gemischt wird.

4. Verfahren nach Anspruch 3, **DADURCH GEKENNZEICHNET, dass** in Schritt b die Biomasse durch Zentrifugation oder Filtration abgetrennt wird.

5. Verfahren nach Anspruch 3, **DADURCH GEKENNZEICHNET, dass** in Schritt c die Alginate durch Ausfällung mit absolutem Ethanol im Verhältnis 1:1 entfernt werden, um sie zu extrahieren, und dann zentrifugiert oder gefiltert werden, um die ausgefallenen Alginate zu trennen.

6. Verfahren nach Anspruch 5, **DADURCH GEKENNZEICHNET, dass** Ethanol durch Verdampfen entfernt wird.

7. Verfahren nach Anspruch 6, **DADURCH GEKENNZEICHNET, dass** der alginatfreie Extrakt gegebenenfalls gefriergetrocknet wird.

8. Verfahren nach Anspruch 7, **DADURCH GEKENNZEICHNET, dass** der gefriergetrocknete alginatfreie Extrakt bei Verwendung in Wasser oder Pufferlösung bei pH 7 resuspendiert wird.

9. Verfahren nach Anspruch 3, **DADURCH GEKENNZEICHNET, dass** zusätzlich eine Fraktionierung durchgeführt wird, bei der die Proteinfraktion durch Verwenden von Ammoniumsulfat im Verhältnis 1:1 Gewicht/Volumen aufgetrennt wird.

10. Zusammensetzung nach Anspruch 1 oder 2 zur Verwendung bei der Behandlung und Vorbeugung von Virusinfektionen.

11. Zusammensetzung zur Verwendung nach Anspruch 10, wobei die Zusammensetzung zur Herstellung eines antiviralen Medikaments verwendet wird.

12. Zusammensetzung zur Verwendung nach Anspruch 11, wobei das Arzneimittel zur topischen Anwendung bestimmt ist.

13. Zusammensetzung zur Verwendung nach Anspruch 11, wobei das Medikament die Vorbeugung oder Behandlung von Haut- oder Mundläsionen ermöglicht, die durch Herpesviren der Unterfamilie *Alphaherpesvirinae* verursacht werden

14. Zusammensetzung zur Verwendung nach Anspruch 11, wobei das Medikament es ermöglicht, die Ästhetik von Haut- oder Mundläsionen zu verbessern, die durch Herpesvirus der Unterfamilie *Alphaherpesvirinae* verursacht werden

15. Zusammensetzung zur Verwendung nach den Ansprüchen 13 und 14, **DADURCH GEKENNZEICHNET, dass** Viren der Unterfamilie *Alphaherpesvirinae* aus Herpes-simplex-Virus Typ 1 (HSV-1), Herpes-simplex-Virus Typ 2 (HSV-2) und Varicella-Zoster-Virus (VZV) ausgewählt sind.

## Revendications

1. Une composition à activité antivirale **CARACTÉRISÉE en ce qu'**elle comprend des extraits hydrosolubles sans alginate de *Durvillaea antarctica,* à une concentration comprise entre 0,05 et 50 mg/mL et un support ou un véhicule.

2. La composition selon la revendication 1, **CARACTÉRISÉE en ce que** l'extrait hydrosoluble sans alginate de *Durvillaea antarctica* ne correspond qu'à la fraction protéique de l'extrait.

3. Procédé de préparation d'une composition à activité antivirale **CARACTÉRISÉ en ce qu'**il comprend les étapes suivantes :
a) mélanger la biomasse broyée de *Durvillaea antarctica* avec de l'eau dans un rapport de 1 à 5 % p/v, à une température comprise entre 15 et 45 °C et agiter pendant 2 à 24 heures ;
b) séparer la biomasse résiduelle du surnageant hydrosoluble ;
c) éliminer les alginates présents dans la solution pour obtenir la phase hydrosoluble, qui constitue l'extrait qui est éventuellement mélangé avec un support ou un véhicule.

4. Procédé selon la revendication 3, **CARACTÉRISÉ en ce que** dans l'étape b, la biomasse est séparée par centrifugation ou filtration.

5. Procédé selon la revendication 3, **CARACTÉRISÉ en ce que** dans l'étape c, les alginates sont éliminés par précipitation avec de l'éthanol absolu dans un rapport éthanol/extrait de 1:1, puis centrifugés ou filtrés pour séparer les alginates précipités.

6. Procédé selon la revendication 5, **CARACTÉRISÉ en ce que** l'éthanol est éliminé par évaporation.

7. Procédé selon la revendication 6, **CARACTÉRISÉ en ce que** l'extrait sans alginate est éventuellement lyophilisé.

8. Procédé selon la revendication 7, **CARACTÉRISÉ en ce que** l'extrait lyophilisé sans alginate est remis en suspension lorsqu'il est utilisé dans de l'eau ou une solution tampon à pH 7.

9. Procédé selon la revendication 3, **CARACTÉRISÉ en ce qu'**un fractionnement est en outre effectué, en séparant la fraction protéique à l'aide de sulfate d'ammonium dans un rapport de 1:1 poids/volume.

10. Composition selon les revendications 1 ou 2 pour une utilisation dans le traitement et la prévention d'une infection virale.

11. Composition pour une utilisation selon la revendication 10, dans laquelle la composition est utilisée pour préparer un médicament antiviral.

12. - Composition pour une utilisation selon la revendication 11, dans laquelle le médicament est pour une application topique.

13. Composition pour une utilisation selon la revendication 11, dans laquelle le médicament permet de prévenir ou de traiter les lésions cutanées ou buccales produites par le virus de l'herpès de la sous-famille *Alphaherpesvirinae.*

14. - Composition pour une utilisation selon la revendication 11, dans laquelle le médicament permet d'améliorer l'esthétique des lésions cutanées ou buccales produites par le virus de l'herpès de la sous-famille *Alphaherpesvirinae.*

15. - Composition pour une utilisation selon les revendications 13 et 14, **CARACTÉRISÉE en ce que** les virus de la sous-famille *Alphaherpesvirinae* sont choisis parmi le virus de l'herpès simplex de type 1 (HSV-1), le virus de l'herpès simplex de type 2 (HSV-2) et le virus varicelle-zona (VZV).
